# EUROPEAN PATENT APPLICATION

(11) **EP 4 821 514 A2**
(43) Date of publication of application: **14.10.2026**
(21) Application number: 26202037.3
(22) Date of filing: 12.02.2021
(51) Int. Cl.: A61L 15/00

(54) **METHOD OF ELECTROSPINNING NATURAL POLYMERS**

(30) Priority: 13.02.2020 IT 202000002833; 05.01.2021 IT 202100000104
(62) Document number(s) of the earlier application(s) in accordance with Art. 76 EPC: 21710569.1 / 4 103 772
(71) Applicant: Bakel S.P.A., 33100 Udine (IT)
(72) Inventor: Gregoris, Raffaella, 33100 Udine (IT); Manfredini, Stefano, 44049 Vigarano Mainarda (FE) (IT); Vertuani, Silvia, 44123 Ferrara (IT)
(74) Representative: Petraz, Gilberto Luigi

(57) **Abstract**

The electrospinning method comprises the steps of providing a composition to be electrospun; providing an electrospinning device comprising an electrospinning head and a collector; applying an electric field between the electrospinning head and the collector; and feeding the composition to be electrospun through the electrospinning head, so that the electric field applied induces the formation of an electrospun fiber.

## Description

### FIELD OF THE INVENTION

The present invention concerns an electrospinning method. More particularly, the invention concerns a method for electrospinning a composition based on a polymer, preferably a biocompatible polymer, to be electrospun.

### BACKGROUND OF THE INVENTION

The electrospinning method in general is known, which allows to obtain continuous fibers with a diameter in the order of a nanometer or micrometer, starting from a composition based on a polymer compound to be electrospun that is subjected to an electric field. Depending on the type of compound treated, the nanofibers obtained can then have applications in any field whatsoever, for example in medicine, military defense, environment, biotechnology, energy or in the cosmetic field.

For environmental reasons, electrospinning tends to be performed using ecological solvents, in particular water. However, electrospinning polymers in pure water is not simple due to its surface tension and the viscosity of the compound that is obtained.

To overcome this problem, attempts have been made to lower the surface tension and viscosity of the water, for example by electrospinning in aqueous solutions of ammonium, therefore in solutions with a high pH, or also in water in the presence of dimethylformamide DMF at 40°C. Experiments have also been performed with hexafluoroisopropanol HFIP or ethanol. By proceeding in this way, however, the ecological aspect is compromised.

There is therefore a need to perfect an electrospinning method which can overcome at least one of the disadvantages of the state of the art.

In particular, one purpose of the present invention is to provide an electrospinning method which is sustainable.

Another purpose of the present invention is to provide an electrospinning method which allows to produce continuous nanofibers, or in any case fibers, with a controlled diameter and free from defects.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the present invention or variants to the main inventive idea.

In accordance with the above purposes, the following describes an electrospinning method which overcomes the limits of the state of the art and eliminates the defects present therein.

In accordance with some embodiments, an electrospinning method is provided which comprises the steps of providing a composition to be electrospun, providing an electrospinning device comprising an electrospinning head and a collector, applying an electric field between the electrospinning head and the collector, and feeding the composition to be electrospun through the electrospinning head, so that the electric field applied induces the formation of an electrospun fiber.

According to some embodiments, the electrospinning head comprises a delivery member which preferably comprises at least one needle.

Preferably, the electric field applied between the electrospinning head and the collector has a potential difference of at least 5 kV, more preferably 8 kV. Even more preferably, the potential difference is comprised between 15 and 40 kV.

Favorably, the electrospinning head and the collector are set in relative motion one with respect to the other, that is, at least one of the two is set in motion.

Advantageously, at least the electrospinning step itself takes place in a controlled and constant atmosphere, more advantageously it takes place in conditions of controlled and constant temperature and relative humidity.

According to some embodiments, the composition comprises a first compound to be electrospun and a spinning promoter. The spinning promoter has the function of facilitating the spinning of the first compound, in particular of establishing the electrospinning method so as to obtain regular fibers.

The first compound to be electrospun is a biocompatible polymer suitable to be electrospun and selected from a group consisting of a first polysaccharide, collagen, gelatin, albumin, elastin and their derivatives. Favorably, the first polysaccharide is selected from a group consisting of xanthan gum, pectin, chitin, chitosan, dextran, carrageenan, guar gum, agar, cellulose derivatives, starch, gelatin, β-glucans, glycosaminoglycans, mucopolysaccharides, water-soluble polysaccharides and their derivatives.

Preferably, the cellulose derivatives are selected from hydroxypropyl methylcellulose HPMC, hydroxypropyl cellulose HPC, hydroxyethyl cellulose HEC, sodium carboxymethyl cellulose Na-CMC. Glycosaminoglycans GAG or mucopolysaccharides can be selected from chondroitin sulfate, dermatan sulfate, heparin, heparan sulfate and hyaluronic acid HA. The water-soluble polysaccharides can be selected from galactomannans, xylans, gum arabic, gum ghatti, glucomannan, acemannan, soluble dietary fiber, glycogen, amylose and polysaccharides derived from plants, bacteria and fungi.

The spinning promoter is a carrier polymer, possibly also without filler, selected from a group consisting of a second polysaccharide, chemically different from the first polysaccharide, possibly in the presence of poly(oxyethylene) PEO. Favorably, the carrier polymer, possibly also without filler, is also biocompatible.

Preferably, the electrospinning promoter is selected from pullulan and alginate, possibly mixed with poly(oxyethylene). The electrospinning promoter can also comprise a mixture of pullulan and alginate. More preferably, the promoter is chemically different from the first compound to be electrospun, that is, the first compound to be electrospun is not pullulan or alginate.

According to some embodiments, the composition also comprises an active ingredient. This active ingredient can, for example, be selected from cosmetic active ingredients, pharmaceutical active ingredients and nutritional active ingredients.

According to some embodiments, it is possible to provide that the active ingredient is not present in the composition to be electrospun, but that it is added to the product obtained by means of the electrospinning.

According to some embodiments, the composition also comprises a stabilizer, suitable to improve the stability of the fibers obtained following the electrospinning. Preferably, the stabilizer is a cross-linkable polymer.

One advantage of the method as above lies in the possibility of producing a cosmetic, pharmaceutical or nutritional product based on natural polymers, with much higher topical concentrations of compound of interest than those obtainable with traditional formulations. This is due to the fact that the compound of interest is integrated precisely into the very structure of the final product. With known methods, it is practically impossible to reach such high concentrations, even with polysaccharides, collagen, gelatin, albumin, elastin and their low molecular weight derivatives, since the viscosity of the product increases too much and it is not possible to exceed 5-10 % by weight. With this composition, it is possible to obtain products that allow to apply concentrations up to 50% by weight of the compound of interest.

### DETAILED DESCRIPTION OF SOME EMBODIMENTS

We will now refer in detail to the possible embodiments of the invention, of which one or more examples are shown in the attached drawings, by way of a non-limiting example. The phraseology and terminology used here is also for the purposes of providing non-limiting examples.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (room temperature) and at atmospheric pressure. All temperatures, unless otherwise indicated, are expressed in degrees Celsius.

All percentages and ratios indicated here are understood to refer to the weight of the total composition (w/w), unless otherwise indicated.

All percentage intervals reported here are supplied with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

Where water is mentioned, we mean distilled water, unless otherwise specified.

The electrospinning method provides to load a composition to be electrospun into a feeder, for example a syringe fed by a volumetric pump. Obviously, the composition is provided in liquid form, as will be explained in more detail below. The feed is performed at a controlled and preferably constant volumetric flow rate, generally comprised between 0.1 and 60 mL/h. The syringe is connected to an electrospinning head containing in particular a delivery member through which the composition is delivered.

Preferably, the delivery member provides a needle, with a known and predetermined diameter, for example comprised between 10G and 40G, preferably 20G and 30G. It is possible to provide that the delivery member comprises several needles, for example two or three or even more, depending on requirements, located side by side to each other. It is also possible to provide two needles located coaxially with respect to each other. According to alternative embodiments, the delivery member provides a metal wire on which the composition is applied, by means of a specific head.

An electric field is applied to the composition, with a potential difference of at least 5 kV, preferably 8 kV. Preferably, the potential difference is comprised between 15 and 40 kV. The electric field is applied by means of a voltage generator, and has the purpose of inducing the formation of an electrospun fiber, for example to form a membrane substrate able to be absorbed by the skin.

The electric field is applied between the electrospinning head, which acts as a positive pole, that is, the pole with the greater potential than the other, and a collector on which the electrospun composition is deposited, the collector acting as a negative pole, that is, the pole with a lower potential than the other. For example, a potential of 0 V is applied to the collector, and a potential equal to the potential difference to be obtained is applied to the electrospinning head, for example 20-25 kV. It is also possible to apply a negative potential to the collector, for example up to -10 kV.

It should be noted that the distance between the delivery head and the collector, to which the potentials are applied in order to create the electric field, is also controlled and preferably kept constant during the electrospinning. Typically, this distance is greater than 2 cm, preferably greater than 5 cm, more preferably it is greater than or equal to 15 cm.

According to some embodiments, the direction of the electric field, which also defines the electrospinning direction of the composition, that is, the direction in which the fiber is delivered at exit from the delivery member, is vertical, from top to bottom. In other words, the electrospinning head is disposed above the collector, and vertically aligned with it.

However, it is possible to provide that this direction of the electric field is vertical from bottom to top (therefore, have a jet of fibers that goes from bottom to top), or that it is horizontal, from right to left or vice versa.

The collector, preferably metallic, can be static, or it can be mobile, for example in the form of an endless belt. In any case, in order to obtain the non-woven fabric form of the fibers, it is advantageous to provide that the electrospinning head is mobile during the electrospinning. For example, the electrospinning head is made to translate back and forth along a linear trajectory.

Advantageously, the collector is coated with a film or a layer of material suitable to receive the fiber that is created, for example in aluminum or PBSA.

The electrified composition at exit from the delivery member solidifies and is deposited on the collector in the form of a continuous fiber, for example creating a film of non-woven fabric, which can form a membrane substrate. Preferably, all the parameters indicated above are kept constant throughout the entire electrospinning step, so as to have a continuous fiber with constant structural characteristics (in particular the diameter).

One example of typical electrospinning conditions are relative humidity (RH) of 20-50%, advantageously of 30-40%, temperature of about 24°C, an electric field of 20-25kV, a flow rate of the feed of the composition of 1 mL/h, diameter of the nozzle equal to 22G and a distance between the delivery head and the collector equal to approximately 20 cm. By "distance between the delivery head and the collector" we mean the minimum distance between these two components.

The application of a strong electric field on the composition based on polymers in solution generates an electrostatic force that prevails over the surface tension of the composition, allowing the formation of a jet that is projected in the direction of the opposite electrode where the collector is located. At exit from the delivery member, the electrically charged solution deforms, creating in a first step a zone with a high-density charge, called a Taylor cone. In a second step, due to the electrostatic repulsion, and because of the evaporation of the solvent, an accelerated and thinned jet is created in which the viscoelastic forces oppose the natural fragmentation of the jet, leading to the solidification of a non-woven fabric of extremely fine fibers on the collector. We wish to reinforce the fact that the fibers created with this method generally have diameters of the order of the nanometer, which are well below the typical diameters of the fibers extruded with mechanical forces.

On the other hand, with regards to the composition to be electrospun, it comprises a first compound to be electrospun, that is, a biocompatible polymer suitable to be electrospun and selected from xanthan gum, pectin, chitin, chitosan, dextran, carrageenan, guar gum, agar, hydroxypropyl methylcellulose HPMC, hydroxypropyl cellulose HPC, hydroxyethyl cellulose HEC, sodium carboxymethyl cellulose Na-CMC, albumin, starch, gelatin, collagen, elastin, β-glucans, chondroitin sulfate, dermatan sulfate, heparin, heparan sulfate, hyaluronic acid HA, galactomannans, xylans, gum arabic, gum ghatti, glucomannan, acemannan, soluble dietary fiber, glycogen, amylose and polysaccharides derived from plants, bacteria and fungi and their derivatives.

These compounds or classes of compounds have, as their property, the possibility of modifying the viscosity of liquids, which makes them suitable to form regular electrospun fibers with good mechanical and absorption properties. They are also all biocompatible, of natural origin, and can be used in the food, pharmaceutical and/or cosmetic sectors.

In particular, xanthan gum, dextran, carrageenan, Na-CMC, starch, gelatin and gum ghatti are used as a thickener, stabilizer and possibly also as a gelling agent in the food sector. In addition, xanthan gum is used as a stabilizer for suspensions and emulsions in the pharmaceutical and cosmetic sectors, guar gum is also used as a thickener and gelling agent in the pharmaceutical and cosmetic sectors, carrageenan is used as an inactive excipient in the pharmaceutical sector. Dextran is also used as a thickener in the pharmaceutical sector.

Chitosan is used in the food sector, in low-calorie diets, and in the pharmaceutical sector as an excipient, in particular for products to be inhaled. Pectin, on the other hand, is used as a gelling agent in the food sector and as a dietary and probiotic agent in the pharmaceutical sector.

Agar, galactomannans and glucomannan are used as a gelling agent in the nutritional sector.

Among cellulose derivatives, HPMC is used as a stabilizer and viscosity regulator in the food sector, and as collyrium or excipient for oral medicines in the pharmaceutical sector. HPC is used as a food additive, and as collyrium or binder for tablets in the pharmaceutical sector. HEC is used as a thickener and gelling agent in the pharmaceutical and cosmetic sectors.

β-glucans are used as dietary fibers, as are xylans. Chondroitin sulfate is used as a food supplement, and also in the treatment of osteoarthritis symptoms. Dermatan sulfate, heparin and heparan sulfate are known as anticoagulants in the pharmaceutical sector.

Gum arabic is used in the food sector as a stabilizing excipient and viscosity modifier. Acemannan, on the other hand, is known in the pharmaceutical sector for its immunostimulant properties.

It should be noted that some of these compounds have their own functions in the cosmetic, pharmaceutical or food sectors, such as for example starch, elastin, hyaluronic acid, heparin, collagen, pectin, β-glucans, chondroitin sulfate, dermatan sulfate, heparan sulfate and their derivatives, among others. It is therefore advantageous to electrospin these compounds, since the application of the corresponding fibers will allow to apply these compounds in higher doses than known solutions, to the advantage of their greater effectiveness.

In the event the first compound to be electrospun is hyaluronic acid, it can be of the linear or cross-linked type, and can have a high mass, for example of the order of a million Dalton or even more, or alternatively have a low mass, typically of the order of 10,000 Daltons or less. It is also possible to provide a mixture of linear hyaluronic acid with cross-linked hyaluronic acid, so as to modulate the rigidity of the yarn that will be obtained, as well as the three-dimensional structure of a film that can be obtained by depositing the yarn on the collector.

The first compound is advantageously diluted in an aqueous or aqueous-based solution, at low concentrations, for example between 0.1% and 10% by weight, preferably 0.5% and 5% by weight, more preferably between 0.6% and 2.5% by weight.

The composition to be electrospun also comprises a spinning promoter which is a carrier polymer without filler, favorably biocompatible. It is selected from alginate, possibly in the presence of PEO, and pullulan. The spinning promoter is preferably pullulan, since it allows to obtain the best results.

In the event that alginate is mixed with PEO, these can be diluted separately in aqueous or aqueous-based solutions, at a concentration comprised between 1% and 40%, preferably between 2% and 30%, more preferably between 4% and 10% by weight. The alginate:PEO mixture, if present, is made in a weight proportion preferably comprised between 5:1 and 1:5, more preferably between 2:1 and 1:2. The best electrospinning results were obtained with proportions equal to 1:1 by weight.

Pullulan can be diluted in an aqueous or aqueous-based solution preferably at a concentration comprised between 1% and 40%, preferably between 3% and 30%, more preferably between 10% and 20% by weight.

The first compound to be electrospun and the promoter are mixed in proportions (first compound):promoter preferably comprised between 10:1 and 1:10, more preferably between 4:1 and 1:7, even more preferably between 3:1 and 1:6 by weight.

The composition also comprises at least one active ingredient, of the pharmaceutical, nutritional and/or cosmetic type.

It should be noted that the active ingredients can have various types of functions, regardless of their field of action.

The cosmetic active ingredient can be of the following types: anti-seborrheic (e.g. sebacic acid, azelaic acid), anti-sebum (e.g. coal powder), antimicrobial (e.g. climbazole, piroctone olamine), antioxidant (e.g. ascorbic acid, tocopherol, coenzyme Q10, resveratrol, glutathione), antiperspirant (e.g. aluminum chlorohydrate, aluminum sesquichlorhydrate), astringent (e.g. Citrus aurantifolia flower extract, calcium lactate), whitener (e.g. glabridin, ammonium persulfate), make-up remover (e.g. sodium cocoyl glutamate), deodorant (e.g. triethyl citrate, zinc ricinoleate), exfoliant (e.g. glycolic acid, malic acid, mandelic acid), flavorings (e.g. citral, honey), fragrance (e.g. d, l-limonene, coumarin), humectant (e.g. glycerin, propanediol), keratolytic (e.g. chloroacetic acid, salicylic acid), moisturizer (e.g. Aloe arborescens leaf extract), perfuming (e.g. geraniol, linalool), emollient (e.g. triolein, squalene), refreshing (e.g. menthol, menthyl lactate), skin moisturizer (e.g. panthenol, allantoin), skin protection (e.g. sphingolipids, zinc oxide), smoothing (e.g. Ricinus communis seed oil), soothing (e.g. Hamamelis virginiana extracts, Chamomile recutica extracts, bisabolol) or tonic (e.g. arnica montana, Capsicum frutescens extract), UV filter (e.g. methylene bis-benzotriazolyl tetramethylbutylphenol, ethylhexyl methoxycinnamate, caffeine, theine, theobromine, theophylline).

The pharmaceutical active ingredient can be of the following types: 5-alpha-reductase inhibitor (e.g. finasteride), 5-aminosalicylates (e.g. mesalamine), 5HT3 receptor antagonist (e.g. ondansetron), ACE inhibitor with calcium channel blocker (e.g. amlodipine/benazepril), ACE inhibitor with thiazides (e.g. hydrochlorothiazide), adamantane antivirals (e.g. amantadine), adrenal corticosteroid inhibitor (e.g. aminoglutethimide), adrenergic bronchodilators (e.g. albuterol), hypertensive urgencies agent (e.g. diazoxide), pulmonary hypertension agent (e.g. treprostinil), aldosterone receptor antagonist (e.g. spironolactone), alkylating agent (e.g. cyclophosphamide), allergens (e.g. house dust mite allergen extracts), alpha-glucosidase inhibitor (e.g. miglitol), amoebicides (e.g. metronidazole), aminoglycosides (e.g. tobramiycin), aminopenicillins (e.g. amoxicillin), aminosalicylates (e.g. aminosalicylic acid), AMPA receptor antagonist (e.g. perampanel), amylin analogues (e.g. pramlintida), analgesics (e.g. acetaminophen), androgenic and anabolic steroids (e.g. testosterone), angiotensin converting enzymes inhibitor (e.g. ramipril), angiotensin II inhibitor with calcium channel blocker (e.g. amlodipine/olmesartan), angiotensin II inhibitor with thiazides (e.g. hydrochlorothiazide/olmesartan), angiotensin receptor blockers (e.g. valsartan), inhibitor of angiotensin and neprilysin receptor blockers (e.g. sacubitril/valsartan), anorectal preparations (e.g. hydrocortisone/pramoxin), anorexiants (e.g. phentermine), antacids (e.g. magnesium hydroxide), anthelmintics (e.g. pyrantel), anti-angiogenic ophthalmic agent (e.g. aflibercept), anti-CTLA-4 monoclonal antibody (e.g. ipilimumab), anti-PD-1 monoclonal antibody (e.g. nivolumab), antiadrenergic agent (central) with thiazides (e.g. hydrochlorothiazide/methyldopa), antiadrenergic agent (peripheral) with thiazides (e.g. polythiazide/prazosin), centrally acting antiadrenergic agent (e.g. guanfacine), peripherally acting antiadrenergic agent (e.g. tamsulosin), antiandrogens (e.g. enzalutamide), antianginal agent (e.g. nitroglycerin), (e.g. dyphylline/guaifenesin), antibiotics (e.g. metronidazole), antibiotics/antineoplastics (e.g. doxorubicin), anticholinergic antiemetics (e.g. diphenhydramine), anticholinergic antiparkinsonian agent (e.g. procyclidine), anticholinergic bronchodilators (e.g. tiotropium), anticholinergics/antispasmodics (e.g. hyoscyamine), anticoagulant agent (e.g. phytonadione), anticonvulsants (e.g. lacosamide), antidepressant (e.g. bupropion), antidiarrheal (e.g. loperamide), antidiuretic hormone (e.g. desmopressin), antidote (e.g. naltrexone dronabinol), antifungal (e.g. griseofulvin), antigonadotropic agent (e.g. g. danazol), antigout agent (e.g. colchicine), antihistamine (e.g. cetirizine), anti-hyperlipidemic agent and combinations (e.g. ezetimibe/simvastatin), antihyperuricemic agent (e.g. febuxostat), antimalarial (e.g. doxycycline), antimalarial combination, antimalarial quinoline (e.g. hydroxychloroquine), antimanic agent (e.g. lithium), antimetabolite (e.g. capecitabine), anti-migraine agent (e.g. rizatriptan), antineoplastic (e.g. isotretinoin), antineoplastic combination (e.g. letrozole/ribociclib), antineoplastic detoxifying agent (e.g. amifostine), antineoplastic interferon (e.g. interferon alfa-2b), antipseudomonal penicillin (e.g. carbenicillin), antipsoriatic agent (e.g. acitretin), antipsychotic agent (e.g. haloperidol), antirheumatic (e.g. adalimumab), antiseptic and germicidal agent, antithyroid agent (e.g. potassium iodide), antitoxin and antiviral (e.g. antivenin (crotalidae) polyvalent), antitussive (e.g. dextromethorphan), antiviral booster (e.g. ritonavir), antiviral interferon (e.g. peginterferon alfa-2a), aromatase inhibitor (e.g. anastrozole), atypical antipsychotic (e.g. aripiprazole), azole antifungal (e.g. fluconazole), bacterial vaccine (e.g. 13-valent pneumococcal vaccine), barbiturate anticonvulsant (e.g. primidone), barbiturate (e.g. phenobarbital), BCR-ABL tyrosine kinase inhibitor (e.g. imatin), benzodiazepine anticonvulsant (e.g. diazepam), benzodiazepine (e.g. clonazepam), beta blocker with thiazides (e.g. bisoprolol/hydrochlorothiazide), beta-lactamase inhibitor (e.g. clavulanic acid), bile acid sequestering agent (e.g. colesevelam), bisphosphonate (e.g. zoledronic acid), BTK inhibitor (e.g. ibrutinib), calcimimetic (e.g. cinacalcet), calcineurin inhibitor (e.g. tacrolimus), calcitonin, calcium channel blocker (e.g. verapamil), anticonvulsant carbamate (e.g. felbamate), carbapenems (e.g. doripenem), carbapenems/beta-lactamase inhibitor (e.g. meropenem/vaborbactam), anticonvulsant carbonic anhydrase inhibitor (e.g. topiramate), carbonic anhydrase inhibitor (e.g. acetazolamide), cardiac stressing agents (regadenoson), cardioselective beta-blockers (e.g. nebivolol), catecholamines (e.g. epinephrine), CD20 monoclonal antibody (e.g. ocrelizumab), CD30 monoclonal antibody (e.g. brentuximab), CD33 monoclonal antibody (e.g. gemtuzumab), CD38 monoclonal antibody (e.g. CD52 monoclonal), (e.g. alemtuzumab), CDK 4/6 inhibitor (e.g. palbociclib), cephalosporins/beta-lactamase inhibitor (e.g. avibactam/ceftazidime), cerumenolytics (e.g. carbamide peroxide), combination of CFTR (e.g. ivacaftor/lumacaftor), CFTR enhancer (e.g. ivacaftor), CGRP inhibitor (e.g. erenumab), chelating agent (e.g. deferasirox), chemokine receptor antagonist (e.g. maraviroc), chloride channel activator (e.g. lubiprostone), cholesterol absorption inhibitor (e.g. ezetimibe), cholinergic agonist (e.g. cevimeline), cholinergic muscle stimulants (e.g. pyridostigmine), cholinesterase inhibitor (e.g. donepezil), central nervous system stimulant (e.g. Phentermine), colony stimulating factor (e.g. Filgrastim), contraceptive (e.g. Levonorgestrel), corticotropin, coumarins and indandione (e.g. Warfarin), cox-2 inhibitor (e.g. Celecoxib), decongestant (e.g. pseudoephedrine), diarylquinoline, dibenzazepine anticonvulsant (e.g. carbamazepine), digestive enzyme (e.g. lactase), dipeptidyl peptidase 4 inhibitor (e.g. sitagliptin), dopaminergic antiparkinsonian agent (e.g. ropinirole), drug used in alcohol dependence (e.g. acamprosate), echinocandin (e.g. caspofungin) inhibitor (e.g. erlotinib), estrogen receptor antagonist (e.g. fulvestrant), estrogen (e.g. estradiol), expectorant (e.g. guaifenesin), factor Xa inhibitor (e.g. rivaroxaban), fatty acid derivative anticonvulsant (e.g. divalproex sodium), fibric acid derivative (e.g. fenofibrate), first generation cephalosporins (e.g. cephalexin), fourth generation cephalosporins (e.g. cefepime), gallstone solubilizing agent (e.g. ursodiol), gamma-aminobutyric acid analogue (e.g. gabapentin), gamma-aminobutyric acid reuptake inhibitor (e.g. tiagabine), general anesthetic (e.g. propofol), GI stimulant (e.g. metoclopramide), glucocorticoids (e.g. budesonide), glucose elevating agent (e.g. glucagon), glycopeptide antibiotic (e.g. vancomycin), glycoprotein platelet inhibitor (e.g. tirofiban), glycylcycline (e.g. tigecycline), gonadotropin releasing hormone (e.g. leuprolide), gonadotropin releasing hormone antagonist (e.g. elagolix), gonadotropin (e.g. chorionic gonadotropin) group I antiarrhythmic (e.g. phenytoin), group II antiarrhythmic (e.g. propranolol), group III antiarrhythmic (e.g. dronedarone), group IV antiarrhythmic (e.g. verapamil), group V antiarrhythmic (e.g. digoxin), growth hormone receptor blocker (e.g. pegvisomant), growth hormone (e.g. somatropin), guanylate cyclase-C agonist (e.g. linaclotide), H. pylori eradication agent (e.g. bismuth subcitrate potassium/metronidazole/tetracyclines), H2 antagonist (e.g. ranitidine), hedgehog pathway inhibitor (e.g. vismodegib), heparin antagonist (e.g. protamine), HER2 inhibitor (e.g. neratinib), herbal product (e.g. 5-hydroxytryptophan, aloe vera), histone deacetylase inhibitor (e.g. romidepsin), hormone/antineoplastic (e.g. medroxyprogesterone), hydantoin anticonvulsant (e.g. phenytoin), hydrazide derivative (e.g. isoniazid), immunoglobulin, impotence agent (e.g. sildenafil), mimetic of incretin (e.g. liraglutide), inotropic agent (e.g. digoxin), insulin and derivatives (e.g. insulin glargine), insulin-like growth factor (e.g. mecasermin), interferon (e.g. interferon beta-1a), interleukin inhibitor (e.g. dupilumab), interleukin (e.g. aldesleukin), iron product (e.g. ferrous sulfate), ketolide (e.g. telithromycin), laxative (e.g. bisacodyl), leprostatic (e.g. clofazimine), leukotriene modifier (e.g. montelukast), lincomycin derivative (e.g. clindamycin), loop diuretic (e.g. furosemide), lysosomal enzyme (e.g. imiglucerase), macrolide (e.g. azithromycin), mast cell stabilizer (e.g. cromolyn), meglitinide (e.g. repaglinide), melanocortin receptor agonist (e.g. bremelanotide), methylxanthine (e.g. theocortic) mineral corticoid (e.g. fludrocortisone), mineral and electrolyte (e.g. citric acid/potassium citrate), various antivirals (e.g. baloxavir marboxil), various anxiolytics, sedatives and hypnotics (e.g. zolpidem), various bone resorption inhibitors (e.g. denosumab), various cardiovascular agents (e.g. midodrine), various agents of the central nervous system (e.g. dalfampridine), various coagulation modifiers (e.g. tranexamic acid), various diuretics (e.g. pamabrom), various agents of the genitourinary tract (e.g. phenazopyridine), various gastrointestinal agents (e.g. misoprostol), various metabolic agents (e.g. burosumab), various respiratory agents (e.g. alpha 1-proteinase inhibitor), various topical agents (e.g. sodium hyaluronate), various vaginal agents (e.g. estradiol), mitotic inhibitor (e.g. vincristine), monoamine oxidase inhibitor (e.g. phenelzine), mouth and throat product (e.g. fluoride), mTOR inhibitor (e.g. everolimus), mucolytic (e.g. acetylcysteine), multikinase inhibitor (e.g. sorafenib), combination of narcotic analgesics (e.g. buprenorphine/naloxone), narcotic analgesic (e.g. fentanyl), natural penicillin (e.g. penicillin v potassium), neuraminidase inhibitor (e.g. oseltamivir), neuronal potassium channel openers (e.g. ezogabine), new generation cephalosporins (e.g. ceftaroline), NHE3 inhibitor (e.g. ceftaroline), nicotinic acid derivative (e.g. ethionamide), NK1 receptor antagonist (e.g. aprepitant), NNRTI (e.g. efavirenz), non-cardioselective beta blocker (e.g. carvedilol), non-sulfonylurea (e.g. metformin), non-steroidal anti-inflammatory drug (e.g. diclofenac), NS5A inhibitor (e.g. daclatasvir), nucleoside reverse transcriptase inhibitor (NRTI) (e.g. tenofovir), nutraceutical product (e.g. omega-3 polyunsaturated fatty acids), oral food supplement (e.g. arginine), other immunostimulants (e.g. glatiramer), other immunosuppressants (e.g. omalizumab), oxazolidinedione anticonvulsant (e.g. trimethadione), oxazolidinone antibiotic (e.g. linezolid), parathyroid hormone and analogues (e.g. teriparatide), PARP inhibitor (e.g. niraparib), PCSK9 inhibitor (e.g. evolocumab), penicillin resistant penicillinase (e.g. oxacillin), peripheral opioid receptor antagonist (e.g. naloxegol), mixed peripheral opioid receptor agonists (egonist/eluxadoline antagonist), peripheral vasodilator (e.g. isoxsuprine), peripherally acting anti-obesity agent (e.g. orlistat), phenothiazine antiemetic (e.g. promethazine), phenothiazine antipsychotic (e.g. prochlorperazine), phenylpiperazine antidepressant (e.g. trazodone), potassium phosphate inhibitor (e.g. trazodone) (e.g. idelalisib), platelet aggregation inhibitor (e.g. aspirin), platelet stimulating agent (e.g. eltrombopag), polyene (e.g. nystatin), potassium-sparing diuretic (e.g. spironolactone), probiotic (e.g. lactobacillus acidophilus), progesterone receptor modulator (e.g. ulipristal), progestin levonorgestrel, prolactin inhibitor (e.g. cabergoline), protease inhibitor (e.g. telaprevir), protease-activated receptor-1 antagonist (e.g. vorapaxar), proteasome inhibitor (e.g. bortezomib), proton pump inhibitor (e.g. omeprazole), psoralen (e.g. methoxsalen), purine nucleoside (e.g. valaciclovir), pyrrolidine anticonvulsant (e.g. levetiracetam), human quinolones (e.g. ciprofloxacin), recombinant human erythropoietin (e.g. epoetin alfa), renin inhibitor (e.g. aliskiren), rifamycin derivative (e.g. rifampicin), salicylate (e.g. aspirin), second generation cephalosporin (e.g. selective cefuroxime receptor), modulator (e.g. ospemifene), selective immunosuppressant (e.g. natalizumab), selective phosphodiesterase-4 inhibitor (e.g. roflumilast), selective serotonin reuptake inhibitor (e.g. escitalopram), serotonin-norepinephrine reuptake inhibitor (e.g. duloxetine), serotonergic neuroenteric (e.g. tegaserod), SGLT-2 inhibitor (e.g. empagliflozin), skeletal muscle relaxant (e.g. onabotulinumtoxinA), smoking quitting agent (e.g. nicotine analog somatostat) (e.g. octreotide), statin (e.g. lovastatin), streptogramin (e.g. dalfopristin/quinupristin), streptomycete derivative (e.g. capreomycin), anticonvulsant succinimide (e.g. ethosuximide), sulfonamide (e.g. sulfamethoxazole), sulphonylurea stimulant (e.g. glimepistole, clomiphene), tetracyclic antidepressant (e.g. mirtazapine), tetracyclines (e.g. minocycline), thiazide diuretic (e.g. hydrochlorothiazide), thiazolidinedione (e.g. pioglitazone), thioxanthene (e.g. thiotixene), third generation cephalosporine (e.g. ceftriaxone), thrombin inhibitor (e.g. dabigatazone), strepolytic (e.g. levothyroxine), TNF alpha inhibitor (e.g. adalimumab), tocolytic agent (e.g. terbutaline), topical acne agent (e.g. tretinoin), topical anesthetic (e.g. lidocaine), topical anti-infectious (e.g. malathion), topical anti-rosacea agent (e.g. ivermectin), topical antibiotic (e.g. silver sulfadiazine), topical antifungal (e.g. econazole), topical antihistamine (e.g. diphenhydramine), topical antineoplastic (e.g. imiquimod), topical anti psoriasis (e.g. tazarotene), topical antivirals (e.g. penciclovir), topical astringent (e.g. hazelnut), topical debridement agent (e.g. collagenase), topical depigmenting agent (e.g. hydroquinone), topical emollient (e.g. emollients), topical keratolytics (e.g. salicylic acid), topical non-steroidal anti-inflammatory (e.g. diclofenac), topical photochemistry (e.g. aminolevulinic acid), topical rubefactive (e.g. menthol), topical steroid (e.g. betamethasone), topical steroid with anti-infectives (e.g. aciclovir/hydrocortisone), transthyretin stabilizer (e.g. tafamidis), anticonvulsant triazine (e.g. lamotrigine), tricyclic antidepressant (e.g. amitriptyline), urea cycle disturbing agent (e.g. sodium phenylbutyrate), urinary anti-infective (e.g. nitrofurantoin), urinary antispasmodic (e.g. amitriptyline) modifier (e.g. potassium citrate), uterotonic agent (e.g. dinoprostone), vaginal anti-infective (e.g. clindamycin), vasodilator (e.g. alprostadil), vasopressin antagonist (e.g. conivaptan), vasopressor (e.g. epinephrine), VEGF/VEGFR inhibitor (e.g. pazopan), viral vaccine, combination of vitamins and minerals, vitamin (e.g. cyanocobalamin), VMAT2 inhibitor (e.g. valbenazine).

The nutritional active ingredient can be of the following types: vitamin (e.g. vitamins A, B, C, D, E, K, folic acid, biotin), mineral (e.g. potassium, chlorine, sodium, calcium, phosphorus, magnesium, iron, zinc, manganese, copper, iodine, chromium, molybdenum, selenium, cobalt, fluoride), amino acid, peptide and protein and their metabolites and derivatives (e.g. essential and branched amino acids, carnosine, enzymes and enzyme complexes, lactoferrin, N-acetylcysteine, proteins animal or vegetable food), fatty acid (e.g. omega-3, omega-6, omega-9 fatty acids), natural product produced using intact sources or substances extracted or derived from plants, animals, algae, fungi, lichens or bacteria (e.g. phytosterol, echinacea, green tea extract, garlic, aloe vera, fish oil, spirulina, chlorella, digestive enzymes derived from mushrooms), sugar and polysaccharides (e.g. mannose, ribose, trehalose, dextrose, glucuronolactone, dextrins), probiotic (e.g. live microorganisms such as Lactobacillus spp, Bifidobacterium spp, Sacc boulardii), prebiotic (e.g. fructans such as fructooligosaccharides and inulins, galactans such as galactooligosaccharides and xylooligosaccharides), antioxidant (e.g. lipoic acid, coenzyme Q10, flavonoids, glutathione, resveratrol, catechins), other substances with a nutritional or physiological effect (e.g. betaine, caffeine, theobromine, theophylline, CDP-choline, choline, creatine, phospholipids, GABA, glucosamine, inositol, melatonin, methylsulfonylmethane, nucleotides, squalene).

The inclusion of the active ingredient in the electrospun fiber can be obtained by co-electrospinning the active ingredient with the first compound. In this case, a mixture can be prepared of the first compound to be electrospun and the electrospinning promoter with the active ingredient, and the mixture obtained is electrospun.

Alternatively, it can be provided to electrospin the first compound on its own, and subsequently to integrate the active ingredient in the fiber obtained. Depending on the applications, it can be provided that the active ingredient is absorbed into the electrospun fiber, or that it is trapped in the three-dimensional structure obtained with the electrospun fiber.

For example, the first compound to be electrospun can comprise a mixture of linear hyaluronic acid with cross-linked hyaluronic acid. Cross-linked hyaluronic acid has the effect of increasing the rigidity of the nanometric fibers obtained, but also of increasing the complexity of the three-dimensional structure of a film achieved by continuously depositing the fibers obtained on several layers. In particular, the presence of cross-linked hyaluronic acid causes the formation of cavities in the film, cavities that allow to house the molecules of active ingredient.

According to another example, the active ingredient is a non-steroidal anti-inflammatory, to be applied for example on a skin burn. It can also be provided to add one or more analgesics as additional active ingredients, in order to reduce the pain caused by the burn. For this type of application, it is particularly advantageous that the first compound is of the type that is regenerating for the skin, such as hyaluronic acid.

The use of the composition according to the invention in the treatment of skin burns is advantageous since it determines a fast absorption of the active ingredient and also of the first compound in the wound. This improves the effectiveness of the treatment.

Advantageously, the active ingredient is present at a concentration comprised between 0.1% and 30% by weight, more preferably between 0.2% and 20% by weight, even more preferably between 0.5% and 10% by weight.

It should be noted that hyaluronic acid, as a compound to be electrospun, is a good candidate to be combined with different active ingredients, of each of the three types indicated above.

For example, among cosmetic active ingredients we can mention the following: anti-seborrheic (sebacic acid, azelaic acid), antioxidants (ascorbic acid, tocopherol, retinol, retinal), anti-stain (glabridin, ammonium persulfate), emollients (Hamamelis virginiana extract, bisabolol) and humectants (e.g. glycerin, propanediol).

Among the preferred nutritional active ingredients are natural products produced using intact sources or substances extracted or derived from plants, animals, algae, fungi, lichens or bacteria (phytosterol, echinacea, green tea extract, garlic, aloe vera, fish oil, spirulina, chlorella, digestive enzymes derived from fungi), vitamins (e.g. vitamins A, B, C, D, E, K, folic acid, biotin) and antioxidants (e.g. lipoic acid, coenzyme Q10, flavonoids, glutathione, resveratrol, catechins).

The most advantageous pharmaceutical active ingredients are androgens and anabolic steroids (e.g. testosterone), anti-CTLA-4 monoclonal antibodies (e.g. ipilimumab), anti-PD-1 monoclonal antibodies (e.g. nivolumab), antianginal agents (e.g. nitroglycerin), anti-asthma combinations (e.g. diphyllin/guaifenesin), antibiotics (e.g. metronidazole), antibiotics/antineoplastics (e.g. doxorubicin), antineoplastics (e.g. isotretinoin) and antineoplastic combinations (e.g. letrozole/ribociclib).

It should be noted that these active ingredients can be advantageously combined with other compounds to be electrospun such as, for example, xanthan gum, guar gum, chondroitin sulfate, collagen or starch.

Another example of composition provides heparin as a compound to be electrospun and a pharmaceutical active ingredient, for example an allergen extract or a platelet stimulating agent such as eltrombopag.

According to some embodiments, the composition also comprises a stabilizer, which is preferably a cross-linkable polymer. One example of a stabilizer is sodium alginate, which is added to pullulan as a promoter. Preferably, the proportion of pullulan:alginate is comprised between 3:1.5 and 3:0.5, more preferably it is equal to 3:1.

### EXAMPLES

Electrospinning tests were performed on examples of composition according to the present description. In the composition examples, the first compound, to be electrospun, is selected from the compounds listed in the table below:

| | |
|---|---|
| HA1 | linear hyaluronic acid with average molecular mass equal to 1.2 MDa |
| HA2 | hyaluronic acid oligomer with average molecular mass lower than 10000 Da |
| HA3 | hyaluronic acid with average molecular mass equal to 50000 Da |
| HA4 | cross-linked hyaluronic acid with average molecular mass comprised between 20 and 3000 kDa |

These compounds are supplied by Esperis S.p.A., Milan, Evonik Degussa Italia, Cremona, and IRALAB S.p.A., Usmate Velate (MI). The molecular masses were determined by means of GPC (Gel Permeation Chromatography).

The electrospinning was performed in a NANON.01A apparatus of the Japanese company Mecc CO. Ltd. The experimental conditions are indicated in each of the examples below.

The fibers produced were characterized by means of scanning electron microscopy. In particular, they were coated with gold using an EMITECHK950x Turbo Evaporator sputter coater, EBSciences, East Granby, CT, and observed with a Cambridge Stereoscan 440 SEM, Cambridge, UK scanning electron microscope.

### Examples of electrospinning of compositions comprising hyaluronic acid as a compound to be electrospun and a mixture PEO:alginate as a spinning promoter

The spinning promoter comprises a mixture of an aqueous solution of alginate at 5% by weight with an aqueous solution of PEO at 5% by weight in a proportion of 1:1. The promoter was then mixed with an aqueous solution of linear hyaluronic acid with an average molecular weight of 1.2 MDa at 0.5% by weight. The promoter:(HA solution) proportion is equal to 5.6:1. The composition was electrospun with relative humidity (RH) comprised between 24% and 29%, at a temperature of 22°C, with an electric field of 20kV, at a volumetric flow rate of the composition at the head equal to 0.7 mL/h, the distance between the spinning head and the collector on which the fiber deposits is equal to 15cm and the needle used being a needle of the 22G type. The fiber obtained is regular and has few defects. The same promoter was mixed with an aqueous solution of hyaluronic acid oligomer at 13% by weight, in promoter:(HA solution) proportion equal to 1:3. The electrospinning of this second example of composition, under the same operating conditions as the first example above, has a very regular and defect-free fiber, with an average diameter comprised between 250 and 350 nm. The fiber obtained completely covered the collector used.

### Examples of electrospinning of compositions comprising hyaluronic acid as a compound to be electrospun and pullulan as a spinning promoter

The pullulan used is of the food grade type, produced by Hayashibara Co., Ltd. Aqueous solutions of pullulan at 10%, 15% or 20% by weight were prepared, and these aqueous solutions of pullulan (spinning promoter) were mixed with aqueous solutions of hyaluronic acid, for the electrospinning.

The table below lists the examples of compositions that were electrospun, as well as the corresponding operating conditions of the electrospinning.

| | Composition | Electrospinning conditions |
|---|---|---|
| 1 | Pullulan 20%:HA3 29% 1:2 | RH 20-30%, 23kV, 0.1µl/min, 15 cm, needle 22G |
| 2 | Pullulan 20%:HA2 29% 1:2 | RH 46%, T=23°C, 23kV, 1ml/h, 15 cm, needle 22G |
| 3 | Pullulan 10%:HA2 23% 1:3 | RH 40%, T=24°C, 23-25kV, 1ml/h, needle 22G, max distance |
| 4 | Pullulan 10%:HA2 15% 1:2 | RH 49%, T=21°C, 23kV, 0.6ml/h, 18 cm, needle 22G, acid pH (between 1.5 and 3) |
| 5 | (pullulan 15%/alginate 5% 3:1):HA2 23% 1:3 | RH 49%, T=21°C, 23kV, 0.6ml/h, 15 cm, needle 22G |
| 6 | Pullulan 10%:HA2 23% 1:3 | RH 49%, T=21°C, 23kV, 0.15ml/h, 15 cm, needle 22G, acid pH (between 1.5 and 3) |
| 7 | Pullulan 10%:HA2 15% 1:2 | RH 40-50%, T=21°C, 23kV, 0.6ml/h, 15 cm, needle 22G, pH = 5.5 |
| 8 | Pullulan 10%:HA2 23% 1:3 | RH 30%, T=22°C, 23kV, 0.5ml/h, 15 cm, needle 22G, pH = 5.5 |

Example 1 resulted in regular fibers, without defects and with an average diameter from 400 to 700 nm. However, little deposit was observed during the test.

In example 2, the fibers obtained are thick, with an average diameter of 10 µm, due to the high viscosity of the electrospun solution.

In example 3, the fibers obtained have an average diameter comprised between 50 nm and 2 µm. It should be observed that with this example the fibers were deposited both on aluminum and also on a film of PBSA.

Examples 4 and 7 (pullulan:HA ratio equal to 1:2), on the one hand, and 6 and 8 (pullulan:HA ratio equal to 1:3), on the other hand, allowed to verify the effect of the proportions between promoter and hyaluronic acid. In example 4, the solution obtained has optimal properties for a good electrospinning, the fibers obtained have an average diameter ranging from 800 nm to 1 µm. For the composition of example 4, which has an acid pH, the pH was increased up to 5.5 (by adding NaOH 1M) thus obtaining the solution of example 7. With the latter, the electrospinning gave regular and uniform fibers with an average diameter smaller than example 4, between 500 and 700 nm.

By increasing the proportion of hyaluronic acid, in example 6 (with acid pH) fibers with a uniform diameter were obtained, with an average value equal to 1-3 µm, while in example 8 (with pH 5.5) the fibers obtained have a non-uniform diameter ranging from 700 nm to 3 µm.

In example 5, an alginate was added to the pullulan as a stabilizer. With a promoter:HA ratio of 1:3, and under the conditions mentioned in the table, thick fibers were obtained, with an average diameter of the order of several microns.

It is clear that modifications and/or additions of parts or steps may be made to the electrospinning method as described heretofore, without departing from the field and scope of the present invention as defined by the claims.

In the following claims, the sole purpose of the references in brackets is to facilitate reading: they must not be considered as restrictive factors with regard to the scope of protection claimed in the specific claims.

## Claims

1. Electrospinning method, comprising:
providing a composition to be electrospun;
providing an electrospinning device comprising an electrospinning head and a collector, between which there is applied an electric field; and
feeding said composition to be electrospun through said electrospinning head, so that the electric field applied induces the formation of an electrospun fiber;
wherein said composition to be electrospun comprises a first compound to be electrospun, an electrospinning promoter and an active ingredient, wherein said first compound to be electrospun is hyaluronic acid; and said electrospinning promoter is selected from alginate, pullulan and a mixture thereof.

2. Method as in claim 1, **characterized in that** the electrospinning head comprises a delivery member which comprises at least one needle.

3. Method as in any claim hereinbefore, **characterized in that** the electric field applied between the electrospinning head and the collector is equal to at least 5kV.

4. Method as in any claim hereinbefore, **characterized in that** the electrospinning head and the collector are set in motion one relative to the other.

5. Method as in any claim hereinbefore, **characterized in that** the electric field is oriented vertically from top to bottom.

6. Method as in any claim hereinbefore, **characterized in that** the proportion between first compound to be electrospun and electrospinning promoter is comprised between 4:1 and 1:7 by weight.

7. Method as in any claim hereinbefore, **characterized in that** it also comprises a stabilizer suitable to stabilize the fiber subsequently obtained.

8. Method as in claim 7, **characterized in that** the stabilizer is a cross-linkable polymer.

9. Method as in claim 7 or 8, **characterized in that** the electrospinning promoter is pullulan, and the stabilizer is an alginate.

10. Method as in any claim hereinbefore, **characterized in that** said composition to be electrospun also comprises at least one active ingredient selected from cosmetic active ingredients, pharmaceutical active ingredients and nutritional active ingredients.

11. Method as in any claim hereinbefore, **characterized in that** the first compound to be electrospun, the electrospinning promoter and the active ingredient are co-electrospun, that is, they are electrospun together.

12. Method as in any claim hereinbefore, **characterized in that** the first compound to be electrospun and the electrospinning promoter are co-electrospun, and the active ingredient is added subsequently to the electrospinning.

13. Method as in claim 10, **characterized in that** the active ingredient comprises a non-steroidal anti-inflammatory and/or one or more analgesics.
